# EUROPEAN PATENT SPECIFICATION

(11) **EP 0 867 172 B1**
(45) Date of publication and mention of the grant of the patent: **25.06.2003**
(21) Application number: 98200681.9
(22) Date of filing: 05.03.1998
(51) Int. Cl.: A61K 7/15, B26B 21/00

(54) **"Preshave lotion"**
Vorrasurwasser
Lotion de pré-rasage

(30) Priority: 19.03.1997 EP 97200809
(43) Date of publication of application: 30.09.1998
(73) Proprietor: Koninklijke Philips Electronics N.V., 5621 BA Eindhoven (NL); Sara Lee / D.E. Household & Body Care Research, 2525 KG Den Haag (NL)
(72) Inventor: Zegers, Cornelis Petrus Gijsbertus Maria, Prof. Holstlaan 6, 5656 AA Eindhoven (NL); Pickert, Adrianus Theodorus, Prof. Holstlaan 6, 5656 AA Eindhoven (NL); Rowland, Mark Louis, Prof. Holstlaan 6, 5656 AA Eindhoven (NL); Reeder, Andries Theunis, Prof. Holstlaan 6, 5656 AA Eindhoven (NL)
(74) Representative: Stolk, Steven Adolph

(56) References cited:
- EP-A- 0 385 312
- WO-A-93/25177
- US-A- 5 449 512

## Description

The invention relates to a preshave lotion for an electric shaver, which comprises a volatile carrier liquid and a polyamide powder.

Such a lotion is applied to the skin prior to shaving with an electric shaver in order to make the skin feel soft and improve the shaving result.

In European patent application EP-A-385312, a preshave lotion is disclosed, which comprises a volatile carrier, such as a lower alcohol, and globular powder of a polymer material such as nylon. Said application also suggests the use of dimethylpolysiloxanes as a volatile carrier. A disadvantage of the use of such siloxanes is the detrimental effect on the electrical contacts within the electric shaver, due to the formation of silicon oxides.

It is an object of the present invention to provide a preshave lotion which is free of polysiloxanes and which improves the feeling of freshness after shaving, the lubrication properties between the shaver head and the skin surface, and the shaving properties of the shaver. Moreover, the preshave lotion according to the invention must be compatible with the plastic, metal and electrical parts of the electric shaver, i.e. the lotion must not attack or corrode such parts.

According to the invention, this object is achieved by a preshave lotion as described in the opening paragraph, which is characterized in that the lotion also comprises 2,2,4,4,6,8,8-heptamethylnonane (or isohexadecane). This non-volatile compound acts as an emollient and is present between the skin surface and the polyamide (nylon) powder during shaving. The addition of said compound to the lotion improves the shaving properties of the shaver, and the refreshing sensation after shaving. Experiments have shown that the combination of polyamide powder and 2,2,4,4,6,8,8-heptamethylnonane in the lotion improves the average length of the shaved-off hears with 28 µm. Surprisingly, other large hydrocarbons, whether branched or not, such as squalane (2,6,10,15,19,23-hexamethyltetracosane) and polysynlane (hydrogenated polyisobutene) do not have the same improved effect.

The volatile carrier liquid to be used in the preshave lotion is not critical, provided that the carrier is cosmetically and dermatologicallly acceptable. Preferably, lower alcohols are used, such as ethanol. The content of the volatile carrier is at least 60% by weight, because of the drying characteristics of the preshave lotion after application to the skin surface.

The polyamide powder, which has a lubricating effect, has a particle size from 1 to 50 µm, preferably between 5 and 20 µm. The content of the polyamide powder is 3 to 10% by weight, preferably about 5%. Below these ranges the lubricating effect is poor, whereas above these ranges the skin feels rough and a whitish matter is left. Polyamide-12 is a particularly suitable powder material which appeared to have better shaving properties than e.g. polyamide-6.

The content of the emollient 2,2,4,4,6,8,8-heptamethylnonane is 2 to 4% by weight. Below 2% the improvement of the shaving properties and the refreshing sensation are poor, whereas above 4% demixing of the lotion takes place, which can be observed by the presence of immiscible beads on the liquid surface.

In order to diminish the irritating effect produced by the volatile liquid carrier, the lotion preferably comprises a relative large amount of (demineralized) water, i.e. 15 to 25 % by weight. Moreover, the presence of water has the additional advantage that the polyamide powder, which precipitates on the bottom of the container, becomes more fluffy and voluminous. This is probably due to the fact that water forces the emollient out from the alcohol phase in to the polyamide powder.

The lotion preferably comprises 2 to 4% by weight of an anti-oxidation agent to prevent corrosion especially of the blades of the shaver. An excellent anti-oxidation agent is sodium lauroyl sarcosinate.

In order to improve the wetting characteristics of the polyamide powder, the lotion preferably comprises D-glucoside.

The lotion may comprise further additives, for example a thickening agent such as hydroxypropylcellulose, an algefacient, such as 1-menthol, fragrances (perfumes), a colorant etc.

The preshave lotion of the present invention can be prepared by mixing the polyamide powder with the volatile carrier liquid, water, and with the above-mentioned additives.

The preshave lotion according to the invention can be packed in a common container, in which the polyamide powder precipitates on the bottom. Preferably the container is shaken well before applying an amount of the lotion to the surface of the skin.

The invention will be elucidated with reference to the embodiment described hereinafter.

### Exemplary embodiment.

A preshave lotion is prepared according to the formulation indicated in the Table.

**TABLE**

| Compound | percentage by weight |
|---|---|
| ethanol | 67.00 |
| demineralised water | 20.05 |
| polyamide-12 powder | 5.00 |
| 2,2,4,4,6,8,8-heptamethylnonane | 3.00 |
| sodium lauroyl sarcosinate | 3.00 |
| D-glucoside, mixed octyl and decyl | 1.00 |
| hydroxypropylcellulose | 0.50 |
| mixture of fragrance materials | 0.30 |
| 1-menthol | 0.15 |
| total | 100.00 |

In order to manufacture a batch of 100 kg, the following compounds are introduced into a stainless steel vessel provided with a stirrer and a homogenizer (Ultra Torax).
- The vessel is filled with 37.000 kg ethanol.
- 0.150 kg menthol is added and stirred until the menthol is dissolved.
- 1.000 kg glucoside is added and stirred until a homogeneous cloudy solution is obtained.
- 5.000 kg polyamide-12 powder (Orgasol 2002 supplied by Elf Atochem S.A.) is slowly added without stirring.
- 3.000 kg 2,2,4,4,6,8,8-heptamethylnonane is added while stirring.
- 3.000 kg sodium lauroyl sarcosinate is added while stirring.
- 0.300 kg perfume is added while stirring.
- 20.050 kg demineralised water is added.
- 0.500 kg hydroxypropylcellulose is added while stirring.
- 30.000 kg ethanol is added, and stirred for one hour.

The preshave lotion according to the invention has excellent shaving characteristics, does not attack plastic, metal and electrical parts of the shaver, and provided a very good refreshing sensation.

## Claims

1. A preshave lotion for an electric shaver, which comprises a volatile carrier liquid and a polyamide powder, **characterized in that** the lotion also comprises 2,2,4,4,6,8,8-heptamethylnonane.

2. A preshave lotion as claimed in Claim 1, **characterized in that** the lotion comprises 2 to 4% by weight 2,2,4,4,6,8,8-heptamethylnonane.

3. A preshave lotion as claimed in Claim 1, **characterized in that** the lotion comprises 3 to 10% by weight polyamide powder.

4. A preshave lotion as claimed in Claim 1, **characterized in that** the lotion comprises 15 to 25% by weight water.

5. A preshave lotion as claimed in Claim 1, **characterized in that** the lotion comprises 2 to 4% by weight of an anti-oxidation agent.

6. A preshave lotion as claimed in Claim 5, **characterized in that** the anti-oxidation agent is sodium lauroyl sarcosinate.

## Patentansprüche

1. Preshavelotion für einen Elektrorasierer, die eine flüchtige Trägerflüssigkeit und ein Polyamidpulver umfasst, **dadurch gekennzeichnet, dass** die Lotion auch 2,2,4,4,6,8,8-Heptamethylnonan enthält

2. Preshavelotion nach Anspruch 1, **dadurch gekennzeichnet, dass** die Lotion 2 bis 4 Gew.-% 2,2,4,4,6,8,8-Heptamethylnonan enthält.

3. Preshavelotion nach Anspruch 1, **dadurch gekennzeichnet, dass** die Lotion 3 bis 10 Gew.-% Polyamidpulver enthält.

4. Preshavelotion nach Anspruch 1, **dadurch gekennzeichnet, dass** die Lösung 15 bis 25 Gew.-% Wasser enthält.

5. Preshavelotion nach Anspruch 1, **dadurch gekennzeichnet, dass** die Lösung 2 bis 4 Gew.-% eines Antioxidationsmittels enthält.

6. Preshavelotion nach Anspruch 5, **dadurch gekennzeichnet, dass** das Antioxidationsmittel Natrium-Lauroyl-Sarconisat ist.

## Revendications

1. Lotion de pré-rasage pour un rasoir électrique, lotion qui est constituée d'un liquide porteur volatil et d'une poudre de polyamide, **caractérisée en ce que** la lotion est également constituée de 2,2,4,4,6,8,8-heptaméthylnonane.

2. Lotion de pré-rasage, selon la revendication 1, **caractérisée en ce que** la lotion est constituée de 2 à 4 % en poids de 2,2,4,4,6,8,8-heptaméthylnonane.

3. Lotion de pré-rasage, selon la revendication 1, **caractérisée en ce que** la lotion est constituée de 3 à 10 % en poids de poudre de polyamide.

4. Lotion de pré-rasage, selon la revendication 1, **caractérisée en ce que** la lotion est constituée de 15 à 25 % en poids d'eau.

5. Lotion de pré-rasage, selon la revendication 1, **caractérisée en ce que** la lotion est constituée de 2 à 4 % en poids d'un agent d'anti-oxydation.

6. Lotion de pré-rasage, selon la revendication 5, **caractérisée en ce que** l'agent d'anti-oxydation est du lauroyl sarcosinate de sodium.
